(19) **Europäisches Patentamt European Patent Office Office européen des brevets**

(11) **EP 4 033 969 B1**

(12) **EUROPEAN PATENT SPECIFICATION**

(45) Date of publication and mention of the grant of the patent:
**29.07.2026  Bulletin 2026/31**

(21) Application number: **20737841.5**

(22) Date of filing: **23.06.2020**

(51) International Patent Classification (IPC):
**A61B 5/0215** (2006.01)    **A61B 5/00** (2006.01)
**A61B 5/021** (2006.01)    **A61M 25/00** (2006.01)
**A61B 5/029** (2006.01)    **A61B 5/024** (2006.01)

(52) Cooperative Patent Classification (CPC):
**A61B 5/02158; A61B 5/02108; A61B 5/7207;**
**A61B 5/7217; A61B 5/7221; A61B 5/7246;**
**A61B 5/7257; A61B 5/7267; A61B 5/7278;**
A61B 5/02405; A61B 5/029

(86) International application number:
**PCT/US2020/039171**

(87) International publication number:
**WO 2021/061242 (01.04.2021 Gazette 2021/13)**

(54) **UNSUPERVISED REAL-TIME CLASSIFICATION FOR ARTERIAL BLOOD PRESSURE SIGNALS**

UNÜBERWACHTE ECHTZEITKLASSIFIZIERUNG FÜR ARTERIELLE BLUTDRUCKSIGNALE

CLASSIFICATION EN TEMPS RÉEL NON SUPERVISÉE POUR SIGNAUX DE PRESSION ARTÉRIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **23.09.2019  US 201962904210 P**

(43) Date of publication of application:
**03.08.2022  Bulletin 2022/31**

(73) Proprietor: **Becton, Dickinson and Company Franklin Lakes, NJ 20006 (US)**

(72) Inventor: **HOLLAND, Alexander Santa Ana, CA 92705 (US)**

(74) Representative: **Eisenführ Speiser Patentanwälte Rechtsanwälte PartGmbB Postfach 31 02 60 80102 München (DE)**

(56) References cited:
**WO-A1-2017/220353**

• **HOLLAND ALEXANDER ET AL: "A Simple Unsupervised, Real-time Clustering Method for Arterial Blood Pressure Signal Classification", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23) - 27 July 2019 (2019-07-27), pages 1509 - 1512, XP033625018, DOI: 10.1109/EMBC.2019.8857110**
• **LI QIAO ET AL: "Artificial arterial blood pressure artifact models and an evaluation of a robust blood pressure and heart rate estimator", BIOMEDICAL ENGINEERING ONLINE, BIOMED CENTRAL LTD, LONDON, GB, vol. 8, no. 1, 8 July 2009 (2009-07-08), pages 13, XP021058637, ISSN: 1475-925X, DOI: 10.1186/1475-925X-8-13**

EP 4 033 969 B1

**Description**

BACKGROUND

The present disclosure relates generally to arterial blood pressure monitoring, and more specifically to classification of sensed arterial blood pressure signals.

Biomedical signal analysis techniques often utilize methods to detect and distinguish abnormal or high noise signals from normal (or expected) signals to reduce output error. Arterial blood pressure (ABP) waveform signals, for example, are often utilized in the determination of hemodynamic parameters, such as cardiac output (CO), vascular resistance, stroke volume, or other hemodynamic parameters that can be used to monitor and/or predict important physiological events, such as hypotension. The existence of noise or other error artifacts in the sensed ABP waveform signal can therefore detrimentally affect the accuracy and reliability of downstream hemodynamic processing operations.

Such ABP waveforms, while exhibiting quasi-periodic cyclic behavior, typically exhibit variations in one or more of amplitude, mean value (offset), and period (stretching) from heartbeat to heartbeat due to natural and expected physiological effects. As such, direct comparison of heartbeat portions of the ABP waveforms to identify noise within the signal is complicated by the naturally-occurring variations in the ABP waveform.

SUMMARY

According to the present invention, a hemodynamic monitor as defined in claim 1, a method as defined in claim 14, and a memory as defined in claim 15 are provided.

In one example, a hemodynamic monitor includes a sensor interface, a beat detection module, a model parameter module, a heartbeat classification module, and a hemodynamic processing module. The sensor interface receives a hemodynamic sensor signal from a hemodynamic sensor. The hemodynamic sensor signal is representative of arterial blood pressure (ABP) of a patient. The beat detection module segregates the received hemodynamic sensor signal into a plurality of heartbeat portions. Each heartbeat portion is representative of the ABP of the patient for one of a plurality of individual heartbeats of the patient. The model parameter module determines, for each of the plurality of heartbeat portions, a set of coefficients representative of frequency components of the respective heartbeat portion to produce a plurality of sets of coefficients. Each set of coefficients includes a same number of coefficients. The model parameter module further normalizes each set of coefficients to produce a plurality of sets of normalized coefficients. The heartbeat classification module determines a set of reference coefficients based on the plurality of sets of normalized coefficients, and provides a quality indicator associated with an individual heartbeat based on a comparison of a set of normalized coefficients for the individual heartbeat to the set of reference coefficients. The hemodynamic processing module uses the quality indicator to produce a modified hemodynamic sensor signal, derives one or more hemodynamic parameters from the modified hemodynamic sensor signal, and outputs the one or more derived hemodynamic parameters.

In another example, a system includes a hemodynamic sensor and a hemodynamic monitor connected to the hemodynamic sensor. The hemodynamic sensor is configured to sense arterial blood pressure (ABP) of a patient. The hemodynamic monitor includes a sensor interface, one or more processors, and computer-readable memory. The sensor interface is configured to receive, from the hemodynamic sensor, a hemodynamic sensor signal representative of the ABP of the patient sensed by the hemodynamic sensor. The computer-readable memory is encoded with instructions that, when executed by the one or more processors, cause the hemodynamic monitor to segregate the received hemodynamic sensor signal into a plurality of heartbeat portions, each heartbeat portion representative of the ABP of the patient for one of a plurality of individual heartbeats of the patient. The computer-readable memory is further encoded with instructions that, when executed by the one or more processors, cause the hemodynamic monitor to determine, for each of the plurality of heartbeat portions, a set of coefficients representative of frequency components of the respective heartbeat portion to produce a plurality of sets of coefficients. Each set of coefficients comprises a same number of coefficients. The computer-readable memory is further encoded with instructions that, when executed by the one or more processors, cause the hemodynamic monitor to normalize each set of coefficients to produce a plurality of sets of normalized coefficients, determine a set of reference coefficients based on the plurality of sets of normalized coefficients, compare a set of normalized coefficients for an individual heartbeat to the set of reference coefficients, and determine, based on the comparing, a quality indicator associated with the individual heartbeat. The computer-readable memory is further encoded with instructions that, when executed by the one or more processors, cause the hemodynamic monitor to use the quality indicator to produce a modified hemodynamic sensor signal, derive one or more hemodynamic parameters from the modified hemodynamic sensor signal, and output the one or more derived hemodynamic parameters.

In another example, a method includes producing, using a hemodynamic sensor, an analog hemodynamic sensor signal representative of arterial blood pressure (ABP) of a patient, sampling the analog hemodynamic sensor signal at a defined sampling rate to produce a sampled hemodynamic sensor signal representative of the ABP of the patient, and segregating the sampled hemodynamic sensor signal into a plurality of heartbeat portions, each heartbeat portion representative of the ABP of the patient for one of a plurality of individual heartbeats of the patient. The method further includes determining, for each of the plurality of heartbeat portions, a set of coefficients representative of frequency components of the respective heartbeat portion to produce a plurality of sets of coefficients. Each set of coefficients comprises a same number of coefficients. The method further includes normalizing each set of coefficients to produce a plurality of sets of normalized coefficients, determining a set of reference coefficients based on the plurality of sets of normalized coefficients, comparing a set of normalized coefficients for an individual heartbeat to the set of reference coefficients, and determining, based on the comparing, a quality indicator associated with the individual heartbeat. The method further includes using the quality indicator to produce a modified hemodynamic sensor signal, deriving one or more hemodynamic parameters from the modified hemodynamic sensor signal, and outputting the derived hemodynamic parameters.

BRIEF DESCRIPTION OF THE DRAWINGS

FIG. 1 is a block diagram illustrating an example hemodynamic monitoring system that implements an unsupervised real-time classification algorithm for classifying arterial blood pressure waveforms received from physiological sensors affixed to a patient.
FIG. 2 is a block diagram illustrating further details of the hemodynamic monitor to process the arterial blood pressure waveforms and provide a quality indicator of individual heartbeat portions.
FIG. 3 is a graph illustrating example arterial blood pressure waveform information that can be segregated into individual heartbeat portions.
FIG. 4 is a flow diagram illustrating example operations of the hemodynamic monitor to determine a set frequency component coefficients representative of the shape of individual heartbeat portions of the arterial blood pressure waveform information.
FIG. 5 is a flow diagram illustrating example operations of the hemodynamic monitor to classify individual heartbeat portions of arterial blood pressure waveforms to provide a quality indicator of the individual heartbeat portion.
FIG. 6 is a flow diagram illustrating example operations of the hemodynamic monitor during an initial learning period of the unsupervised real-time classification algorithm.

DETAILED DESCRIPTION

As described herein, a hemodynamic monitoring system implements an unsupervised real-time classification algorithm that classifies individual heartbeat portions of a sensed arterial blood pressure (ABP) waveform to provide a quality indicator of the associated heartbeat portion. The classification algorithm produces, on a heartbeat-by-heartbeat basis, a set of normalized frequency component coefficients that represent a shape of each individual heartbeat portion independent of scaling, mean value (offset), and period (stretching) of the respective heartbeat portion. Normalized sets of frequency component coefficients for newly-sensed individual heartbeat portions are compared to a set of reference coefficients developed by the classification algorithm. A quality indicator for each heartbeat portion is provided for use in downstream processing of the ABP waveform. The quality indicator, in certain examples, classifies each individual heartbeat as belonging to one of two classes, i.e., a class that is qualified for use in downstream hemodynamic processing, and a class that is unqualified for use in the downstream processing. Hemodynamic parameters (e.g., cardiac output, vascular resistance, stroke volume, or other parameters) are therefore derived from those heartbeat portions that are classified as qualified for use in the downstream processing. Portions of the ABP waveform that are classified as unqualified for use in the downstream processing are ignored or otherwise excluded from the hemodynamic processing operations. As such, a hemodynamic monitoring system implementing techniques of this disclosure can increase the accuracy and reliability of hemodynamic parameters derived from the sensed ABP waveforms of a patient.

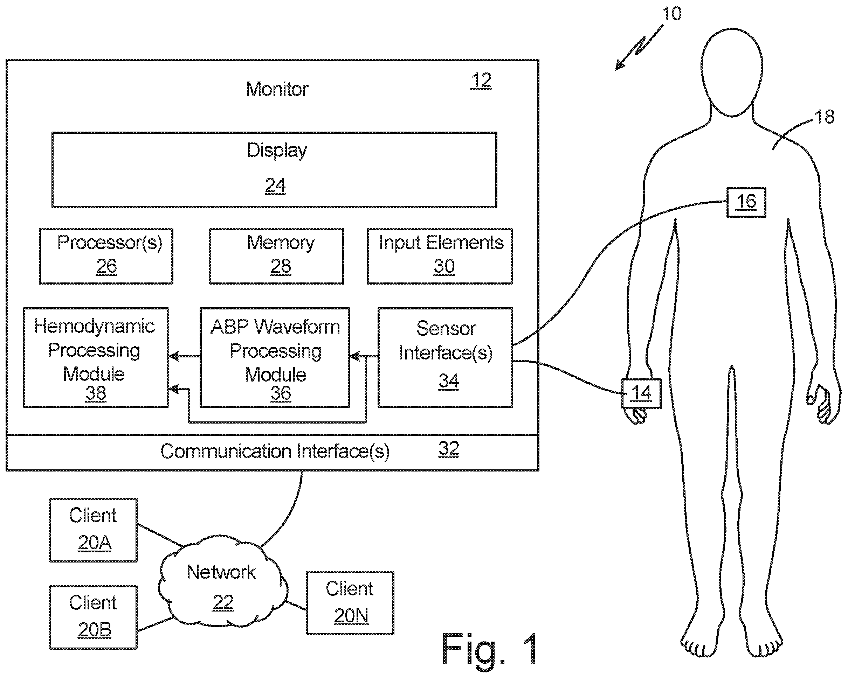

FIG. 1 is a block diagram of hemodynamic monitoring system 10 including hemodynamic monitor 12 that implements an unsupervised real-time classification algorithm for processing and classifying arterial blood pressure (ABP) waveforms received from physiological sensors 14 and/or 16 affixed to patient 18. As illustrated in FIG. 1, system 10 can further involve client devices 20A-20N that are communicatively connected to hemodynamic monitor 12 via communications network 22. Hemodynamic monitor 12 includes display 24, processor(s) 26, computer-readable memory 28, input element(s) 30, communication interface(s) 32, sensor interface(s) 34, arterial blood pressure (ABP) waveform processing module 36, and hemodynamic processing module 38.

As illustrated in FIG. 1, physiological sensors 14 and 16 can be affixed to patient 18 to sense ABP waveforms of patient 18 and deliver the sensed ABP waveform signals to hemodynamic monitor 12. Physiological sensor 14 can include, e.g., one or more cuffs (e.g., one or more finger cuffs) or other peripheral artery pressure sensors that can be used to sense peripheral artery blood pressure. For example, physiological sensor 14 can perform real-time finger pressure measurements using a volume clamp method at a sampling rate of, e.g., 1000 Hertz (Hz) to provide a sensed ABP waveform to hemodynamic monitor 12.

Physiological sensor 16 can be, e.g., a pulmonary artery catheter (PAC), such as a Swan-Ganz catheter. Such a catheter can be inserted into a pulmonary artery of patient 18 to detect direct, simultaneous measurement of pressures in the right atrium, the right ventricle, the pulmonary artery, and the filling pressure of the left atrium of patient 18 by way of a thermal filament located on the catheter and using thermodilution principles. Though the example of FIG. 1 illustrates two physiological sensors 14 and 16 that sense ABP waveform information of patient 18, it should be understood that in other examples, a single physiological sensor (i.e., one of physiological sensors 14 and 16) can be utilized to sense the ABP waveform and provide the waveform information to hemodynamic monitor 12. In yet other examples, more than two physiological sensors can be used to sense and provide the ABP waveform information.

Hemodynamic monitor 12, as illustrated in FIG. 1, can include one or more sensor interfaces 34 that communicate with physiological sensors 14 and 16 to receive information from (and, in certain examples, transmit information to) one or more of physiological sensors 14 and 16. Sensor interfaces 34 can communicate with physiological sensors 14 and 16 via wired or wireless connections, or both. Physiological sensors 14 and 16 can produce analog hemodynamic sensor signals representative of the ABP waveform of patient 18. Sensor interfaces 34 can, in certain examples, include analog-to-digital converters or other equivalent discrete or integrated logic circuitry to sample the analog sensor signals at a defined (e.g., consistent) rate, such as one hundred Hz or other sampling rates, to produce discrete-time ABP waveform information.

As illustrated in FIG. 1, hemodynamic monitor 12 can further include display 24, one or more processors 26, computer-readable memory 28, and input elements 30. Display 24 can be an electronic visual display that renders a graphical user interface for displaying information that characterizes the measured data received from physiological sensors 14 and 16. Such information can take the form of, e.g., waveforms, numerical indications, categorical indications, alerts, or other information. Input elements 30 can include various physical and/or graphical control elements, such as physical and/or virtual buttons, knobs, sliders, or other control elements that can be manipulated (e.g., by a user) to affect operation of hemodynamic monitor 12. For instance, input elements 30 can include physical and/or graphical control elements that enable user interaction with hemodynamic monitor 12 to select the information that is graphically rendered at display 24, to configure operational parameters of hemodynamic monitor 12 (or other components of system 10), to acknowledge alarms generated by hemodynamic monitor 12, or to otherwise affect operation of components of system 10. In some examples, display 24 can be a touch-sensitive display that renders one or more graphical control elements of input elements 30 and enables user interaction in the form of gesture input (e.g., touch gestures, swipe gestures, pinch gestures, or other gesture input).

One or more processors 26, in one example, are configured to implement functionality and/or process instructions for execution within hemodynamic monitor 12. For instance, one or more processors 26 can be capable of processing instructions stored in computer-readable memory 28. Examples of one or more processors 26 can include any one or more of a microprocessor, a controller, a digital signal processor (DSP), an application specific integrated circuit (ASIC), a field-programmable gate array (FPGA), or other equivalent discrete or integrated logic circuitry.

Computer-readable memory 28 can be configured to store information within hemodynamic monitor 12 during operation. Computer-readable memory 28, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). Computer-readable memory 28, in some examples, is described as volatile memory, meaning that computer-readable memory 28 does not maintain stored contents when power to hemodynamic monitor 12 is turned off. Examples of volatile memories can include random access memories (RAM), dynamic random access memories (DRAM), static random access memories (SRAM), and other forms of volatile memories. In some examples, computer-readable memory 28 is used to store program instructions for execution by one or more processors 26. Computer-readable memory 28, in one example, is used by software or applications running on hemodynamic monitor 12 (e.g., a software program implementing aspects of ABP waveform processing module 36 and/or hemodynamic processing module 38) to temporarily store information during program execution. Computer-readable memory 28, in some examples, also includes non-volatile storage elements. Examples of such non-volatile storage elements can include, but are not limited to, magnetic hard discs, optical discs, flash memories, or forms of electrically programmable memories (EPROM) or electrically erasable and programmable (EEPROM) memories.

As illustrated in FIG. 1, hemodynamic monitor 12 can include one or more communication interfaces 32 that enable direct or indirect communication with one or more remote client computing systems, such as client devices 20A-20N that are communicatively coupled with hemodynamic monitor 12 via wired and/or wireless communications network 22. For instance, client devices 20A-20N, which can include any number of client devices, can access information generated by hemodynamic monitor 12 to remotely monitor hemodynamic parameters of patient 18, transmit commands or other information to hemodynamic monitor 12, or otherwise interact with components of system 10.

Sensor interface 34, as illustrated in FIG. 1, provides the ABP waveform information (i.e., the analog and/or discrete-time waveforms) to ABP waveform processing module 36. In some examples, such as the illustrated example of FIG. 1, sensor interface 34 also provides the ABP waveform information to hemodynamic processing module 38, though in other examples, the ABP waveform information can be provided to hemodynamic processing module 38 via ABP waveform processing module 36.

The sampled ABP waveform information received by ABP waveform processing module 36 via sensor interface 34 represents the sensed ABP waveform of patient 18 in discrete time (i.e., sampled at a consistent rate, such as one hundred Hz). Such ABP waveforms can be considered quasi-periodic, in that the waveform exhibits clearly delineated cyclic behavior, but with beat-to-beat variation in shape and period. Certain variations in amplitude and period modulations of the ABP waveform are considered normal, caused by physiological factors such as respiratory modulation and baroreflex regulation of the human body. Abnormal variations in amplitude and period of the ABP waveform can, for example, be introduced by: mechanical coupling of physiological sensors 14 and/or 16 due to patient movement, surgical tubing dynamics, and vessel clamping; electrical interferences from electrocautery instruments or analog circuit nonlinearity distortions; and physiological malfunctions such as ectopic beats, irregular heart patterns, or atrial/ventricular fibrillation. Such abnormal variations in the ABP waveform signal can represent noise artifacts that negatively impact downstream processing of the ABP waveform for hemodynamic monitoring and parameter estimation.

As is further described below, ABP waveform processing module 36 implements an unsupervised real-time classification algorithm that provides a quality indicator associated with individual heartbeats that is used by hemodynamic processing module 38 for the determination and monitoring of hemodynamic parameters, such as cardiac output (CO), vascular resistance, stroke volume, or other hemodynamic parameters. In some examples, the quality indicator for each individual heartbeat classifies the heartbeat as either qualified for use in downstream processing or unqualified for use in downstream processing. In other examples, the quality indicator provides a quantitative indication of an extent by which the individual heartbeat deviates from a representation of a reference heartbeat of patient 18 developed by the unsupervised classification algorithm.

As described herein, ABP waveform processing module 36 segregates the received ABP waveform signal into heartbeat portions, each portion representative of the ABP of patient 18 for an individual heartbeat. For each individual heartbeat portion, ABP waveform processing module 36 determines a set of coefficients representative of frequency components of the individual heartbeat portion, such as by using a finite Fourier series expansion of the discrete-time representation of the individual heartbeat portion. ABP waveform processing module 36 generates a same number of coefficients for each heartbeat portion, irrespective of a period of the individual heartbeat portion. As such, each set of frequency coefficients is a non-uniform discrete-time vector parameter sequence that is representative of a shape of the individual heartbeat portion independent of the period of the individual heartbeat portion. ABP waveform processing module 36 further normalizes each set of frequency component coefficients, thereby producing a set of normalized coefficients for each individual heartbeat portion that represents a shape of the heartbeat portion independent of scaling, mean value (offset), and period (stretching) of the respective heartbeat portion.

The normalized sets of frequency coefficients for individual heartbeat portions are provided as input to an unsupervised real-time clustering algorithm implemented by ABP waveform processing module 36, which determines a set of reference coefficients using multiple sets of the normalized coefficients (i.e., associated with multiple individual heartbeat portions). The set of reference coefficients represents a center reference for a "normal" cluster group or class of normalized coefficient sets (i.e., representative of a typical or otherwise expected heartbeat of the patient).

Normalized coefficients for a newly-sensed individual heartbeat portion are compared to the set of reference coefficients for generation of a quality indicator of the newly-sensed individual heartbeat portion. The quality indicator can, in certain examples, classify the newly-sensed individual heartbeat portion as either qualified for use in downstream processing by hemodynamic processing module 38 or unqualified for use in the downstream processing. In other examples, the quality indicator can provide a quantitative measure of a deviation of the newly-sensed individual heartbeat portion from the set of reference coefficients (i.e., the center reference cluster group or class).

ABP waveform processing module 36 provides the quality indicator (e.g., the classification) associated with each individual heartbeat portion to hemodynamic processing module 38. Hemodynamic processing module 38 utilizes the quality indicator for determination and/or monitoring of hemodynamic parameters based on the ABP waveform signal. For example, hemodynamic processing module 38 can use the quality indicator to produce a modified hemodynamic sensor signal from which one or more hemodynamic parameters are derived and output for, e.g., display or further analysis operations. For instance, in some examples, hemodynamic processing module 38 can produce the modified hemodynamic sensor signal to include individual heartbeat portions that are indicated as qualified for use in downstream processing. Hemodynamic processing module 38 can produce the modified hemodynamic sensor signal to not include individual heartbeat portions that are indicated as unqualified for use in the downstream processing, thereby decreasing the existence of noise or other error artifact within the ABP signal used for hemodynamic parameter monitoring and/or processing.

Accordingly, hemodynamic monitor 12 implementing techniques described herein can provide a quality indicator of individual heartbeat portions of a sensed ABP signal of patient 18. The quality indicator can be used to classify the individual heartbeat portions of the sensed ABP waveform as either qualified for use in downstream processing or unqualified for use in the downstream processing, thereby increasing accuracy and reliability of the hemodynamic parameters derived from the ABP waveform signal. The classification algorithm utilizes frequency parameters representative of the shape of the ABP waveform that are independent of scaling, mean value, and period of each individual heartbeat portion of the ABP waveform, thereby increasing consistency of classification and decreasing operational complexity associated with time-domain analysis of individual heartbeat portions associated with naturally-occurring variances in the period of the individual heartbeats. Moreover, the unsupervised nature of the clustering algorithm enables effective classification of individual heartbeats in real-time based on previously-sensed ABP waveforms of the patient without requiring large amounts of reference data that may be difficult to obtain and which may be unrepresentative of the typical ABP waveform of the particular patient. As such, techniques described herein can increase the accuracy and reliability of hemodynamic parameters derived from sensed ABP waveforms of a patient.

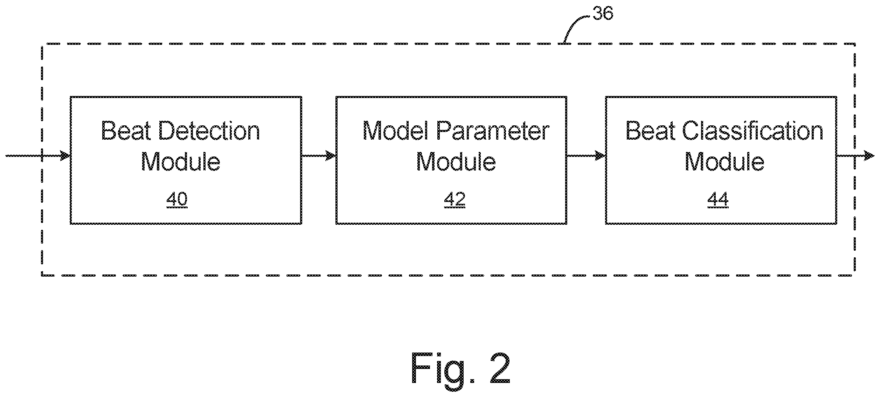

FIG. 2 is a block diagram illustrating further details of ABP waveform processing module 36 to provide a quality indicator of individual heartbeat portions of sensed ABP waveforms. As illustrated in FIG. 2, ABP waveform processing module 36 includes beat detection module 40, model parameter module 42, and beat classification module 44.

Beat detection module 40 receives, as input, the ABP waveform signal sensed by one or more of physiological sensors 14 and 16 (FIG. 1). In some examples, the received ABP waveform signal can be a discrete-time signal sampled at a consistent rate (e.g., one hundred Hz) by sensor interface 34 (FIG. 1). In other examples, the received ABP waveform signal can be an analog ABP waveform signal, in which case beat detection module 40 can sample the received analog signal to produce a discrete-time ABP waveform.

Beat detection module 40 identifies heartbeat portions of the received ABP waveform signal, each heartbeat portion representative of the ABP waveform of patient 18 (FIG. 1) for an individual heartbeat. For example, beat detection module 40 can identify individual heartbeat portions of the received ABP waveform based on a maximum derivative or other feature(s) of the ABP waveform signal that identify a portion of the received ABP waveform signal corresponding to an individual heartbeat from the beginning of systolic upstroke to the end of diastolic pressure. Various detection algorithms for identifying individual heartbeats within an ABP waveform are known in the art, and in general, beat detection module 40 can utilize any detection algorithm suitable for identifying individual heartbeat portions of a received ABP waveform signal.

Beat detection module 40 segregates the received ABP waveform signal into individual heartbeat portions that are provided, along with the discrete-time ABP waveform, to model parameter module 42. For example, beat detection module 40 can identify a beginning index and an ending index of the discrete-time ABP waveform signal for each individual heartbeat portion. Such indices (i.e., beginning and ending indices) can take the form of unique ordered indices of the discrete-time ABP waveform that identify the time-sequential order of sampled values within the discrete-time ABP waveform, absolute time values associated with each of the beginning and ending indices, relative time values associated with each of the beginning and ending indices, or other indications that identify the individual heartbeat portions within the discrete-time ABP waveform.

Model parameter module 42 receives the discrete-time ABP waveform and the indications of the locations of individual heartbeat portions within the discrete-time ABP waveform from beat detection module 40. As is further described below, model parameter module 42 determines, for each individual heartbeat portion, a set of coefficients representative of frequency components of the individual heartbeat portion. The set of coefficients can be determined, e.g., using a finite Fourier series expansion that produces frequency coefficients representative of a shape of the individual heartbeat portion. Rather than utilize a constant period for the Fourier series expansion, model parameter module 42 determines the frequency coefficients based on a period of the individual heartbeat portion. Model parameter module 42 can determine the period of the individual heartbeat portion based on the indications of the beginning and end of the individual heartbeat portion within the discrete-time ABP waveform provided by beat detection module 40. For instance, model parameter module 42 can identify the period of each individual heartbeat portion by multiplying the number of samples included in the individual heartbeat portion by a defined sampling rate at which the discrete-time ABP waveform was generated. In other examples, such as when beat detection module 40 provides an indication of a beginning and ending time (i.e., relative or absolute time) of each individual heartbeat portion, model parameter module 42 can determine the period of the individual heartbeat portion as the difference between the ending time and the beginning time indicated by beat detection module 40.

Model parameter module 42 determines the set of frequency component coefficients for each individual heartbeat portion based in part on the period of the individual heartbeat portion. Each set of frequency component coefficients includes a same number of coefficients. As such, each set of frequency component coefficients represents a shape of the associated individual heartbeat portion using a same number of frequency component coefficients, independent of the period of the respective heartbeat portion.

Model parameter module 42 normalizes each set of frequency component coefficients, thereby producing a normalized set of frequency component coefficients for each individual heartbeat portion that is independent of scaling, mean value, and period of the respective heartbeat portion. The normalized sets of frequency component coefficients are provided by model parameter module 42 to beat classification module 44.

Beat classification module 44 implements an unsupervised real-time clustering algorithm that classifies each individual heartbeat portion based on a comparison of the received normalized set of frequency component coefficients to a reference set of frequency component coefficients. The reference set of frequency component coefficients represents a center reference for a "normal" cluster group or class of normalized coefficient sets, and can be developed by beat classification module 44 during an initial learning operational phase, as is further described below. Beat classification module 44 compares, on a heartbeat-by-heartbeat basis, the normalized set of reference coefficients for a newly-sensed individual heartbeat portion to the set of reference frequency component coefficients to produce a quality indicator associated with the individual heartbeat portion.

In some examples, beat classification module 44 produces a quality indicator identifying two sets (or classes) of individual heartbeat portions, namely: individual heartbeat portions associated with frequency component coefficients that are similar to (e.g., within a threshold deviation from) the reference set of frequency component coefficients and are classified as qualified for use in downstream processing by hemodynamic processing module 38 (FIG. 1); and individual heartbeat portions associated with frequency component coefficients that are dissimilar to (e.g., exceeding a threshold deviation from) the reference set of frequency component coefficients and are classified as unqualified for use in downstream processing by hemodynamic processing module 38 (FIG. 1). In other examples, beat classification module 44 can produce a quality indicator identifying more than two sets or classes of individual heartbeat portions, such as by comparing a deviation between the frequency component coefficients for individual heartbeat portions and the set of reference frequency component coefficients to a plurality of threshold deviations, each threshold deviation quantifying an extent by which the frequency component coefficients for the individual heartbeat portions deviate from the set of reference frequency components.

Beat classification module 44 provides, for each individual heartbeat portion, an indication of the individual heartbeat portion (e.g., a location within the discrete-time ABP waveform signal of the individual heartbeat portion) and a quality indicator associated with the individual heartbeat portion to hemodynamic processing module 38 (FIG. 1). Accordingly, ABP waveform processing module 36 identifies a quality indicator (e.g., a classification) for each individual heartbeat portion that is used to increase accuracy of downstream hemodynamic parameter determinations.

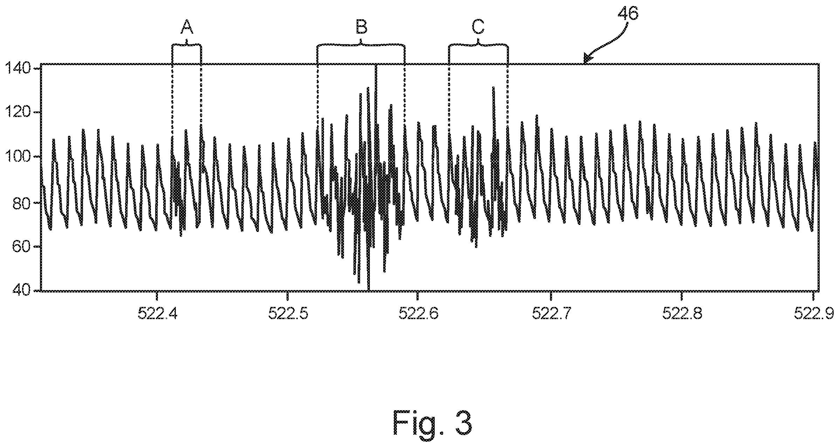

FIG. 3 is a graph illustrating example ABP waveform 46 that can be segregated into individual heartbeat portions by beat detection module 40 of FIG. 2. The example graph of ABP waveform 46 illustrates ABP of, e.g., patient 18 (FIG. 1) in pressure units of millimeters of Mercury (mmHg) as plotted against time in units of minutes.

As illustrated in FIG. 3, ABP waveform 46 includes a pressure waveform that can be segregated into multiple individual heartbeat portions from the beginning of systolic upstroke to the end of diastolic pressure. As further illustrated, ABP waveform 46 includes regions A, B, and C, which in this example, include spurious pressure measurements or other error artifacts that result in signal noise in the form of alterations to the period, amplitude, and shape of ABP waveform 46. Accordingly, the downstream use of individual heartbeat portions identified by beat detection module 40 (FIG. 2) within regions A, B, and C can result in decreased accuracy of the downstream hemodynamic parameter determinations (e.g., by hemodynamic processing module 38 of FIG. 1).

As is further described below, beat classification module 44 (FIG. 2) produces a quality indicator for individual heartbeat portions of ABP waveform 46, including those individual heartbeat portions identified within regions A, B, and C. The quality indicator can, in certain examples, classify the individual heartbeat portions as either qualified for use in the downstream processing or unqualified for use in the downstream processing. In the example of FIG. 3, one or more individual heartbeat portions identified within regions A, B, and C can be classified by beat classification module 44 as unqualified for use in the downstream processing, and individual heartbeat portions identified within ABP waveform 46 outside of regions A, B, and C can be classified by beat classification module 44 as qualified for use in the downstream processing. As such, as is further described below, beat classification module 44 can provide a quality indicator associated with individual heartbeat portions of an ABP waveform that can be used to decrease an amount of noise or other error artifact within the ABP waveform that is used for hemodynamic parameter determinations or other monitoring techniques.

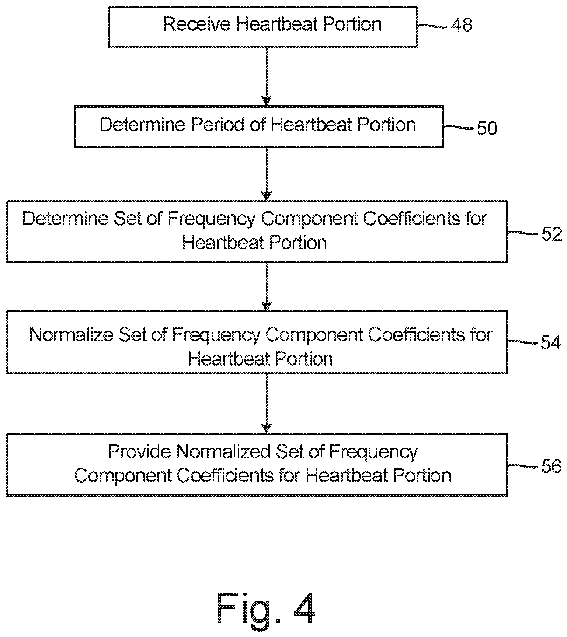

FIG. 4 is a flow diagram illustrating example operations of model parameter module 42 of FIG. 2 to determine a set of frequency component coefficients representative of the shape of individual heartbeat portions. For purposes of clarity and ease of discussion, the example operations are described below within the context of hemodynamic monitoring system 10 of FIG. 1 and ABP waveform processing module 36 described above with respect to FIGS. 1 and 2.

An individual heartbeat portion of an ABP waveform signal is received (Step 48). For example, model parameter module 42 can receive, from beat detection module 40, an indication of a location (e.g., via discrete-time indices) of an individual heartbeat portion within a sensed ABP waveform signal. The period of the individual heartbeat portion is determined (Step 50). For instance, model parameter module 42 can receive, from beat detection module 40, indications of a beginning and an end of the individual heartbeat portion within the discrete-time **ABP** waveform signal, such as an index within the discrete-time signal corresponding to the beginning of the individual heartbeat portion (i.e., a beginning of systolic upstroke) and an index corresponding to the end of the individual heartbeat portion (i.e., an end of diastolic pressure).

A set of frequency component coefficients is determined for the individual heartbeat portion (Step 52). For example, a finite Fourier series expansion of the received individual heartbeat portion can be expressed according to the following equation:

$$s_n(t) = w_n(t) + \sum_{l=-M}^{M} c_l[n] \, e^{i\frac{2\pi l(t-t_S[n])}{T[n]}} \qquad \text{(Equation 1)}$$

where:

n is an index number of the individual heartbeat portion within the ABP waveform;
$s_n(t)$ is the ABP waveform signal for the $n^{th}$ heartbeat index number;
$w_n(t)$ represents model error for the $n^{th}$ heartbeat index number;
$l$ is an index ranging from $-M$ to $+M$;
M corresponds to the highest frequency coefficients;
$c_l[n]$ are the frequency component coefficients for the $n^{th}$ heartbeat portion;
$t_s[n]$ is the starting time within the ABP waveform of the $n^{th}$ heartbeat portion; and
$T[n]$ is the period of the $n^{th}$ heartbeat portion.

Accordingly, the frequency component coefficients representative of the shape of an $n^{th}$ individual heartbeat portion can be determined according to the following equation:

$$c_l[n] = \frac{1}{T[n]} \int_0^{T[n]} s_n(t) e^{i\frac{-2\pi l(t-t_S[n])}{T[n]}} dt \qquad \text{(Equation 2)}$$

where:

n is the index number of the individual heartbeat portion within the ABP waveform;
$l$ is the index ranging from $-M$ to $+M$;
$c_l[n]$ are the frequency component coefficients for the $n^{th}$ heartbeat portion;
$T[n]$ is the period of the $n^{th}$ heartbeat portion;
$s_n(t)$ is the ABP waveform signal for the $n^{th}$ heartbeat index number; and
$t_s[n]$ is the starting time within the ABP waveform of the $n^{th}$ heartbeat portion.

Model parameter module 42 therefore determines the set of coefficients representative of frequency component magnitude and phase of the received individual heartbeat portion. The determined set of frequency component coefficients for an $n^{th}$ individual heartbeat portion can be expressed in vector notation according to the following equation:

$$c[n] = [c_1[n] \quad ... \quad c_M[n]]^T \qquad \text{(Equation 3)}$$

In some examples, model parameter module 42 can insert the mean of the determined coefficients and the period of the individual heartbeat portion into the frequency component coefficient vector. In such examples, the determined set of frequency component coefficients for an $n^{th}$ individual heartbeat portion can be expressed in vector notation according to the following equation:

$$c[n] = [c_0[n], c_1[n], ..., c_{M+1}[n]]^T \qquad \text{(Equation 4)}$$

where $c_0[n]$ is the mean of coefficients $c_1[n]$ through $c_M[n]$, and $c_{M+1}[n]$ is the period of the $n^{th}$ individual heartbeat portion. Though in the example Equation 4 above, the mean of the frequency component coefficients $c_1[n]$ through $c_M[n]$ is inserted at a beginning index $c_0[n]$ and the period of the $n^{th}$ individual heartbeat portion is inserted at an ending index $c_{M+1}[n]$, it should be understood that the mean and period can be inserted at any defined location of the set of frequency component coefficients.

The set of frequency component coefficients for the individual heartbeat portion is normalized (Step 54). For example, model parameter module 42 can normalize the set of frequency component coefficients representative of the shape of the individual heartbeat portion represented above in vector notation with respect to Equation 3 using a division by a 2-norm of the set of frequency component coefficients, or other normalization technique. In some examples, such as when model parameter module 42 represents the set of frequency component coefficients in vector notation according to Equation 4 above (i.e., including the mean of the frequency component coefficients and the period of the individual heartbeat portion), model parameter module 42 can normalize the set of frequency component coefficients utilizing only those elements of the set that are representative of the shape of the individual heartbeat portion (i.e., excluding the elements corresponding to the mean of the coefficients and the period of the individual heartbeat portion). For instance, model parameter module 42 can normalize the set of frequency component coefficients for an $n^{th}$ individual heartbeat portion according to the following equation:

$$c_s[n] = \left[ c_0[n], \frac{c_1[n]}{\|c[n]\|_s}, ..., \frac{c_M[n]}{\|c[n]\|_s}, T[n] \right] \qquad \text{(Equation 5)}$$

where:

$c_s[n]$ is the set of normalized coefficients for the $n^{th}$ heartbeat portion;
$c_0[n]$ is the mean of the frequency component coefficients prior to normalization;
$T[n]$ is the period of the $n^{th}$ heartbeat portion; and
$\|c[n]\|_s$ is the 2-norm of the set of frequency coefficients $[c_1[n] ... c_M[n]]$.

The 2-norm of the set of frequency component coefficients can be determined according to the following equation:

$$\|c[n]\|_s = \sqrt{\sum_{i=1}^{M} c_i^2[n]} \qquad \text{(Equation 6)}$$

The normalized set of frequency component coefficients for the individual heartbeat portion is provided to beat classification module 44 (Step 56). For example, model parameter module 42 can provide the set of frequency component coefficients, $c_s[n]$, described above with respect to Equation 5 as the set $\left[ c_0[n], \frac{c_1[n]}{\|c[n]\|_s}, ..., \frac{c_M[n]}{\|c[n]\|_s}, T[n] \right]$, to beat classification module 44 for classification and determination of a quality indicator associated with the individual heartbeat portion.

Though the example of FIG. 4 describes a single iteration of model parameter module 42 to determine frequency component coefficients representative of the shape of the individual heartbeat portion, it should be understood that the example operations of FIG. 4 can be performed iteratively, on a heartbeat-by-heartbeat basis, to provide multiple sets of normalized frequency component coefficients for multiple individual heartbeats for classification by beat classification module 44.

Model parameter module 42 determines each set of frequency component coefficients (and hence, each set of normalized frequency component coefficients) as having a same number of coefficients, such as 128 coefficients or other numbers of coefficients. Each set of frequency component coefficients is determined based on the period of the associated individual heartbeat portion. As such, even though the periods of individual heartbeat portions can differ due to expected physiological behavior, model parameter module 42 provides a set of normalized frequency coefficients for each individual heartbeat portion that is representative of the shape of the individual heartbeat portion using a same number of frequency component coefficients to facilitate comparison of the shape of multiple individual heartbeat portions or other downstream processing/classification by beat classification module 44. Moreover, the normalized sets of frequency component coefficients represent the shape of the individual heartbeat portions independent of scaling (amplitude), mean value (offset), and period (stretching) of the respective individual heartbeat portions, thereby further facilitating the comparison and classification of the individual heartbeat portions by accounting for natural (or normal) deviations in amplitude, offset, and period of the individual heartbeat portions due to physiological factors such as respiratory modulation and baroreflex regulation of the human body.

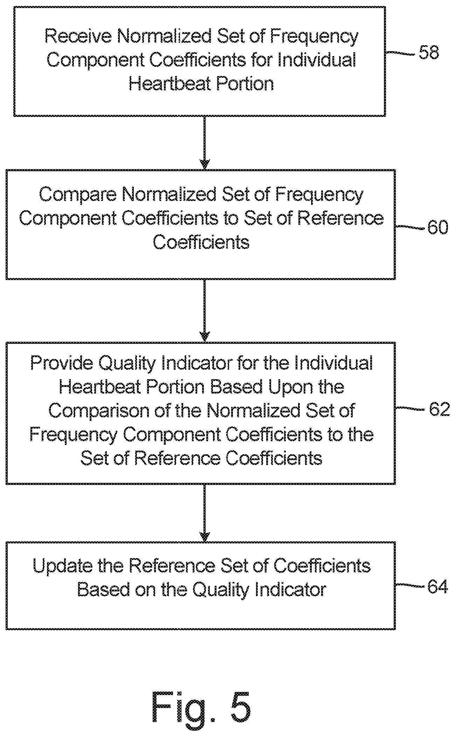

FIG. 5 is a flow diagram illustrating example operations of beat classification module 44 of FIG. 2 to classify individual heartbeat portions of ABP waveforms to provide a quality indicator associated with the individual heartbeat portion. For purposes of clarity and ease of discussion, the example operations are described below within the context of hemodynamic monitoring system 10 of FIG. 1 and ABP waveform processing module 36 described above with respect to FIGS. 1 and 2.

A normalized set of frequency component coefficients for an individual heartbeat portion is received (Step 58). For instance, beat classification module 44 can receive the set of normalized frequency component coefficients, $c_s[n]$,

which can be described in vector notation as, e.g., $\left[ c_0[n], \frac{c_1[n]}{\|c[n]\|_s}, \ldots, \frac{c_M[n]}{\|c[n]\|_s}, T[n] \right]$, from model parameter module 42.

The normalized set of frequency component coefficients for the individual heartbeat portion is compared to a set of reference frequency component coefficients (Step 60). For instance, beat classification module 44 can generate a center reference for a "normal" (i.e., representative of a typical or otherwise expected heartbeat of the patient) cluster group or class using, e.g., an iterative estimate for the mean (or other central tendency) of received sets of normalized frequency component coefficients, as is further described below.

Beat classification module 44 can utilize the determined center reference as the reference set of normalized frequency component coefficients, and can compare the received set of normalized frequency component coefficients for the individual heartbeat portion to the set of reference frequency component coefficients to identify an extent by which the received set of normalized frequency component coefficients (and hence, the shape of the individual heartbeat portion) deviates from the set of reference frequency component coefficients (i.e., representative of an "average" or "normal" heartbeat for the patient). For example, beat classification module 44 can compare the received set of normalized frequency component coefficients for an $n^{th}$ individual heartbeat portion to the set of reference frequency component coefficients according to the following equation:

$$D(c_s[n], \bar{c}_s[n]) = \|c_s[n] - \bar{c}_s[n]\|_W \qquad \text{(Equation 7)}$$

where:

$c_s[n]$ is the received set of normalized frequency component coefficients;
$\bar{c}_s[n]$ is the set of reference normalized frequency component coefficients; and
$W$ is a weighting vector represented as $[w_0, w_1, \ldots, w_{M+1}]^T$.

Accordingly, beat classification module 44 can determine a deviation (or distance) between the received set of normalized frequency component coefficients and the set of reference frequency component coefficients using a weighted Euclidean norm, as expressed in Equation 7 above, though other distance functions or deviation techniques are possible.

Individual elements of the weighting vector $W$, expressed as $[w_0, w_1, \ldots, w_{M+1}]^T$, can be selected empirically to suit the particular application. In some examples, for instance, individual heartbeats having irregularly long or short periods due to physiological conditions can be considered "normal" (or conforming), in which case decreasing the value of the element of the weighting vector $w_{M+1}$ (i.e., corresponding to the period of the individual heartbeat portion) is appropriate. In examples where individual heartbeats having irregularly long or short periods due to physiological conditions are considered "abnormal" (or nonconforming), increasing the value of the element of the weighting vector $w_{M+1}$ is appropriate. Similarly, the mean value of the frequency component coefficients for the individual heartbeat portion represented by normalized frequency component coefficient $c_0[n]$ and weighted by weighting vector element $w_0$ can change due to "normal" (or expected) physiological causes. As such, decreasing the value of weighting vector element $w_0$ can decrease the effect of (or penalize) unexpected or otherwise rapidly changing values of the mean coefficient value $c_0[n]$. Increasing the value of weighting vector element $w_0$ can increase (or emphasize) the effect of the value of the mean coefficient value $c_0[n]$.

A quality indicator associated with the individual heartbeat portion is provided based on the comparison of the set of normalized frequency component coefficients to the set of reference frequency component coefficients (Step 62). For instance, in examples where beat classification module 44 classifies the individual heartbeat portion as belonging to one of two separate classes (or groups), namely either a class that is qualified for use in downstream processing or a class that is unqualified for use in downstream processing, beat classification module 44 can compare the determined distance $D(c_s[n], \bar{c}_s[n])$ or other deviation between the set of normalized frequency component coefficients and the set of reference frequency component coefficients to a threshold deviation according to the following equation:

$$D(c_s[n], \bar{c}_s[n]) < \beta \qquad \text{(Equation 8)}$$

where $\beta$ is a threshold deviation parameter.

The threshold deviation parameter $\beta$ can be an empirically determined value, such as a value between 0 and 1 (or other ranges), that segregates individual heartbeat portions associated with normalized frequency component coefficients that are similar to the set of reference frequency component coefficients (i.e., less than the threshold deviation parameter $\beta$) from individual heartbeat portions associated with normalized frequency component coefficients that are dissimilar to the set of reference frequency component coefficients (i.e., greater than the threshold deviation parameter $\beta$). Beat classification module 44 can, in such examples, provide a quality indicator representing the classification of the individual heartbeat portion, such as a value of 0 (or other defined value) in response to determining that the deviation (or distance) between the set of normalized frequency component coefficients and the set of reference frequency component coefficients is less than threshold deviation parameter (e.g., $\beta$), or a value of 1 (or other defined value) in response to determining that the deviation is not less than (e.g., greater than or equal to) the threshold deviation parameter (e.g., $\beta$).

Accordingly, beat classification module 44 can, in certain examples, classify each individual heartbeat portion as belonging to one of two classes, i.e., a class that is qualified for use in downstream processing (i.e., similar to the reference heartbeat) or a class that is unqualified for use in the downstream processing (i.e., dissimilar to the reference heartbeat). The quality indicator can be provided to, e.g., hemodynamic processing module 38 (FIG. 1) for use in hemodynamic parameter determinations or other hemodynamic monitoring techniques. For instance, hemodynamic processing module 38 can utilize the individual heartbeat portions that are classified as qualified for use in the downstream processing with hemodynamic parameter determinations, such as determination of cardiac output (CO), vascular resistance, stroke volume, or other hemodynamic parameters. Hemodynamic processing module 38 can refrain from utilizing (e.g., by discarding or otherwise refraining from utilizing) the individual heartbeat portions that are classified as unqualified for use in the downstream processing. For example, hemodynamic processing module 38 can discard (or ignore) portions of the received ABP waveform signal that are indicated as unqualified for use with the downstream processing, thereby assembling a usable ABP waveform signal from the ordered combination of individual heartbeat portions that are indicated as qualified for use in the downstream processing.

In some examples, beat classification module 44 can classify individual heartbeat portions as belonging to one of three or more classes, such as three or more classes that represent an extent by which the set of normalized frequency component coefficients for the individual heartbeat portion deviates from the set of reference frequency component coefficients. In such examples, hemodynamic processing module 38 can utilize the classification of each individual heartbeat portion during hemodynamic parameter determinations, such as by weighting the contribution of each defined class differently. For instance, hemodynamic processing module 38 can apply a greatest weight to individual heartbeat portions associated with normalized frequency component coefficients that are classified as most similar to (i.e., having a least deviation from) the set of reference frequency component coefficients. Similarly, hemodynamic processing module 38 can apply a least weight to individual heartbeat portions associated with normalized frequency component coefficients that are classified as least similar to (i.e., having a greatest deviation from) the set of reference frequency component coefficients.

In certain examples, beat classification module 44 can provide the deviation (or distance) of the set of normalized frequency component coefficients from the set of reference frequency component coefficients (e.g., the value of the distance $D(c_s[n], \bar{c}_s[n])$) determined according to Equation 7 above to hemodynamic processing module 38 for use in the downstream processing. For instance, hemodynamic processing module 38 can utilize the actual value of the determined deviation as a coefficient (or gain) that affects an amount of contribution of the individual heartbeat portion to the hemodynamic parameter determinations. In yet other examples, beat classification module 44 can provide to hemodynamic processing module 38 the difference between the deviation ($D(c_s[n], \bar{c}_s[n])$) and the threshold deviation value ($\beta$). Accordingly, beat classification module 44 can provide a quality indicator associated with the individual heartbeat portion that is usable by hemodynamic processing module 38 during the downstream processing of the ABP waveform to determine one or more hemodynamic parameters.

The reference set of coefficients is updated based on the quality indicator (Step 64). For example, beat classification module 44 can determine the reference set of frequency component coefficients using, e.g., an iterative estimate for the mean (or other central tendency) of multiple received sets of normalized frequency component coefficients, as is further described below. Beat classification module 44, in some examples, utilizes the received set of normalized frequency component coefficients in the iterative estimate for the mean to update the set of reference frequency component coefficients in response to determining that the individual heartbeat portion is classified as qualified for use in the downstream processing (e.g., the deviation between the set of frequency component coefficients for the individual heartbeat portion and the set of reference frequency component coefficients is less than the threshold deviation parameter $\beta$). Beat classification module 44 can refrain from using the received set of normalized frequency component coefficients for the individual heartbeat portion in the iterative estimate for the mean (i.e., to determine the set of reference frequency component coefficients) in response to determining that the individual heartbeat portion is classified as unqualified for use in the downstream processing (e.g., the deviation between the set of frequency component coefficients for the individual heartbeat portion and the set of reference frequency component coefficients is greater than or equal to the threshold deviation parameter $\beta$).

As such, beat classification module 44 implements an unsupervised real-time classification algorithm that provides, on a heartbeat-by-heartbeat basis, a quality indicator associated with each individual heartbeat portion. The quality indicator is used by hemodynamic processing module 38 to identify those portions of the ABP waveform that are suitable for use in the downstream hemodynamic parameter determinations, thereby reducing the amount of noise or other artifact that is included in the ABP waveform signal from which the hemodynamic parameters are derived. The unsupervised classification algorithm updates the center reference (i.e., the set of reference frequency component coefficients) based on the classification, thereby adapting to the ABP waveform signal that is sensed by physiological sensors (e.g., one or more of physiological sensors 14 and 16 of FIG. 1) to further increase accuracy of the classification scheme.

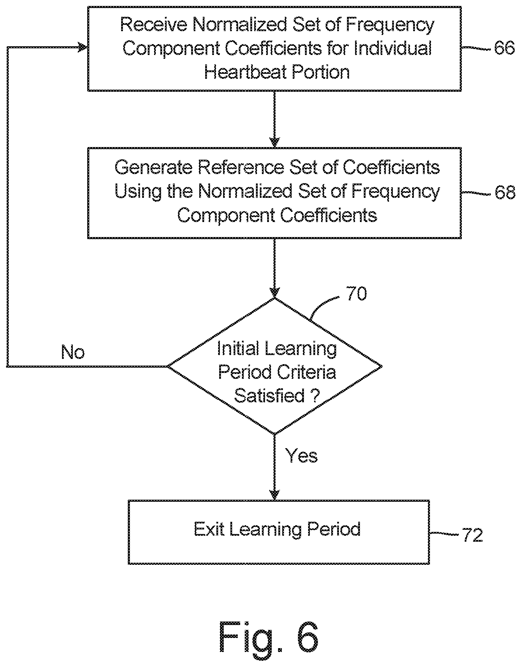

FIG. 6 is a flow diagram illustrating example operations of beat classification module 44 of FIGS. 2 and 5 during an initial learning period of the unsupervised real-time classification algorithm. The example operations of FIG. 6 can be performed by beat classification module 44 during, e.g., an initial stage of monitoring to develop the reference set of frequency component coefficients that are utilized by the real-time classification algorithm for classification of individual heartbeat portions. For purposes of clarity and ease of discussion, the example operations are described below within the context of hemodynamic monitoring system 10 of FIG. 1 and ABP waveform processing module 36 described above with respect to FIGS. 1 and 2.

A normalized set of frequency component coefficients for an individual heartbeat portion is received (Step 66). For example, beat classification module 44 can receive the set of normalized frequency component coefficients $c_s[n]$, described above with respect to Equations 5 and 6, from model parameter module 42.

A reference set of frequency component coefficients is generated using the set of normalized frequency component coefficients (Step 68). For instance, beat classification module 44 can determine the reference set of frequency component coefficients for an $n^{th}$ individual heartbeat portion using an iterative estimate for the mean of received sets of normalized frequency component coefficients according to the following equation:

$$\bar{c}_s[n] = (1 - \alpha)\bar{c}_s[n - 1] + \alpha c_s[n] \qquad \text{(Equation 9)}$$

where:

n is the index number of the individual heartbeat portion within the ABP waveform;
$\bar{c}_s[n]$ is the reference set of frequency component coefficients; and
$\alpha$ is an iterative averaging parameter.

The value of the iterative averaging parameter $\alpha$ can be empirically determined, such as a value greater than zero and less than one, which controls how much weight is given to the past estimate versus the latest input. The value of the iterative averaging parameter $\alpha$ therefore controls how fast the set of reference frequency component coefficients $\bar{c}_s[n]$ adapts to a changing heartbeat shape (e.g., a changing heartbeat shape due to "normal" or expected physiological factors). Smaller values of the iterative averaging parameter $\alpha$ provide a smaller weight to new values, thereby causing slower adaptation of the set of reference frequency component coefficients $\bar{c}_s[n]$. Larger values of the iterative averaging parameter $\alpha$ provide greater weight to new values, thereby causing faster adaptation of the set of reference frequency component coefficients $c_s[n]$.

It is determined whether an initial learning period criterion is satisfied (Step 70). For instance, in some examples, beat classification module 44 can determine that the initial learning period criterion is satisfied in response to receiving a threshold number of sets of normalized frequency component coefficients, such as ten sets, twenty sets, or other numbers of sets of normalized frequency component coefficients.

In other examples, beat classification module 44 can determine that the initial learning period criterion is satisfied in response to determining that a threshold number of sets of normalized frequency component coefficients have been received that satisfy a threshold standard deviation criterion. For instance, beat classification module 44 can determine an iterative estimate for the standard deviation of the set of reference frequency component coefficients $\bar{c}_s[n]$ according to the following equation:

$$\hat{c}_\sigma[n] = (1 - \alpha)\hat{c}_\sigma[n - 1] + \alpha\sqrt{|c_s[n] - \bar{c}_s[n]|^2} \qquad \text{(Equation 10)}$$

where:

$n$ is the index number of the individual heartbeat portion within the ABP waveform;
$\hat{c}_\sigma[n]$ is iterative estimate of the standard deviation;
$\alpha$ is an iterative averaging parameter;
$c_s[n]$ is the set of normalized frequency component coefficients; and
$\bar{c}_s[n]$ is the set of reference frequency component coefficients.

Beat detection module 44 can, in some examples, compare a 2-norm or other normalization of the standard

deviation $\hat{c}_\sigma[n]$ to a threshold standard deviation criterion to determine whether the initial learning period criterion is satisfied. The threshold standard deviation criterion can be an empirically determined value, such as a value equal to half of the threshold minimum number of sets of normalized frequency component coefficients (e.g., ten, twenty, or another threshold value).

Beat detection module 44 can, in such examples, determine that the initial learning period criteria is satisfied in response to determining that the minimum number of sets of normalized frequency component coefficients has been received (e.g., ten, twenty, or other minimum number of sets) and that the standard deviation of the determined set of reference frequency component coefficients $\bar{c}_s[n]$ satisfies the threshold standard deviation criterion.

In response to determining that the initial learning period criteria is not satisfied ("NO" branch of Step 70), beat classification module 44 can continue to receive normalized sets of frequency component coefficients for individual heartbeat portions (Step 66) during the initial learning period. In response to determining that the initial learning period criteria is satisfied ("YES" branch of Step 70), beat classification module 44 can exit the initial learning period phase (Step 72), and can enter a real-time classification phase to classify normalized sets of frequency component coefficients to provide associated quality indicators, as was described above with respect to the example operations of FIG. 5.

As such, hemodynamic monitoring system 10 implementing techniques of this disclosure can implement an unsupervised real-time classification algorithm that classifies and provides a quality indicator associated with individual heartbeat portions of a sensed ABP signal of a patient. The classification algorithm utilizes frequency parameters representative of the shape of the ABP waveform that are independent of scaling, mean value, and period of each individual heartbeat portion of the ABP waveform, thereby increasing consistency of classification and decreasing operational complexity associated with time-domain analysis of individual heartbeat portions associated with naturally-occurring variances in the period of the individual heartbeats. Techniques described herein can therefore increase the accuracy and reliability of hemodynamic parameters derived from sensed ABP waveforms of a patient.

**Claims**

1.  A hemodynamic monitor (12) comprising:

    a sensor interface (34) that receives a hemodynamic sensor signal from a hemodynamic sensor (14, 16), the hemodynamic sensor signal representative of arterial blood pressure, hereinafter ABP of a patient (18);
    a beat detection module (40) that segregates the received hemodynamic sensor signal into a plurality of heartbeat portions, each heartbeat portion representative of the ABP of the patient for one of a plurality of individual heartbeats of the patient;
    a model parameter module (42) that:

    determines, for each of the plurality of heartbeat portions, a set of coefficients representative of frequency components of the respective heartbeat portion to produce a plurality of sets of coefficients, each set of coefficients comprising a same number of coefficients; and
    normalizes each set of coefficients to produce a plurality of sets of normalized coefficients;
    a heartbeat classification module (44) that:

    determines a set of reference coefficients based on the plurality of sets of normalized coefficients; and
    provides a quality indicator associated with an individual heartbeat based on a comparison of a set of normalized coefficients for the individual heartbeat to the set of reference coefficients; and
    a hemodynamic processing module (38) that:

    uses the quality indicator to produce a modified hemodynamic sensor signal;
    derives one or more hemodynamic parameters from the modified hemodynamic sensor signal; and
    outputs the one or more derived hemodynamic parameters.

2.  The hemodynamic monitor of claim 1, wherein the quality indicator classifies the individual heartbeat as either qualified for use in downstream processing or unqualified for use in the downstream processing.

3.  The hemodynamic monitor of claim 1, wherein the heartbeat classification module (44) generates the quality indicator based on an extent by which the set of normalized coefficients for the individual heartbeat deviates from the set of reference coefficients.

4.  The hemodynamic monitor of claim 3, wherein the heartbeat classification module (44) determines the extent by which the set of normalized coefficients for the individual heartbeat deviates from the set of reference coefficients as a vector norm of the difference between the set of normalized coefficients for the individual heartbeat and the set of reference coefficients.

5. The hemodynamic monitor of claim 1, wherein the model parameter module (42) determines, for each of the plurality of heartbeat portions, the set of coefficients representative of frequency components of the respective heartbeat portion by:

identifying, for each of the plurality of heartbeat portions, a period of the respective heartbeat portion; and determining the set of coefficients for the respective heartbeat portion based on the identified period of the respective heartbeat portion.

6. The hemodynamic monitor of claim 5, wherein the model parameter module (42) determines the set of coefficients for the respective heartbeat portion based on the identified period of the respective heartbeat portion using a Fourier series expansion of the respective heartbeat portion as a function of the identified period of the respective heartbeat portion.

7. The hemodynamic monitor of claim 6, wherein the model parameter module (42) determines the set of coefficients using the Fourier series expansion of the respective heartbeat portion as the function of the identified period of the respective heartbeat portion according to the equation:

$$c_l[n] = \frac{1}{T[n]} \int_0^{T[n]} s_n(t) e^{i\frac{-2\pi l(t - t_s[n])}{T[n]}} dt$$

wherein $n$ is an index number identifying the respective heartbeat portion within the hemodynamic sensor signal; wherein $l$ is an index ranging from a negative value of a highest frequency coefficient to a positive value of the highest frequency coefficient; wherein $c_l[n]$ are the set of coefficients for the respective heartbeat portion; wherein $T[n]$ is the identified period of the respective heartbeat portion; wherein $s_n(t)$ is the hemodynamic sensor signal; and wherein $t_s[n]$ is a starting time within the hemodynamic sensor signal of the respective heartbeat portion.

8. The hemodynamic monitor of claim 1, wherein the model parameter module (42) normalizes each set of coefficients to produce the plurality of sets of normalized coefficients by dividing each coefficient from a respective set of coefficients by a 2-norm of the respective set of coefficients.

9. The hemodynamic monitor of claim 1, wherein the heartbeat classification module (44) determines the set of reference coefficients based on the plurality of sets of normalized coefficients using an iterative estimate for a mean of the sets of normalized coefficients.

10. The hemodynamic monitor of claim 9, wherein the heartbeat classification module (44) determines the set of reference coefficients using the iterative estimate for the mean of the sets of normalized coefficients according to the equation:

$$\bar{c}_s[n] = (1 - \alpha)\bar{c}_s[n - 1] + \alpha c_s[n]$$

wherein $\bar{c}_s[n]$ is the set of reference coefficients for a heartbeat index n; and wherein $\alpha$ is an iterative averaging parameter.

11. The hemodynamic monitor of claim 10, wherein $\alpha$ is a number greater than zero and less than one.

12. The hemodynamic monitor of claim 1, wherein the quality indicator classifies the individual heartbeat as either qualified for use in downstream processing or unqualified for use in the downstream processing; and wherein the hemodynamic processing module (38) uses the quality indicator to produce the modified hemodynamic sensor signal by:

producing the modified hemodynamic sensor signal to include the individual heartbeat in the modified hemodynamic sensor signal in response to determining that the quality indicator classifies the individual heartbeat as qualified for use in the downstream processing; and

producing the modified hemodynamic sensor signal to not include the individual heartbeat in the modified hemodynamic sensor signal in response to determining that the quality indicator classifies the individual heartbeat as unqualified for use in the downstream processing.

**13.** A system (10) comprising:

a hemodynamic sensor (14, 16) configured to sense arterial blood pressure, hereinafter ABP of a patient; and

a hemodynamic monitor (12) according to one of the preceding claims connected to the hemodynamic sensor.

**14.** A computer-implemented method comprising:

producing (48), using a hemodynamic sensor, an analog hemodynamic sensor signal representative of arterial blood pressure, hereinafter ABP of a patient;

sampling (48) the analog hemodynamic sensor signal at a defined sampling rate to produce a sampled hemodynamic sensor signal representative of the ABP of the patient;

segregating (50) the sampled hemodynamic sensor signal into a plurality of heartbeat portions, each heartbeat portion representative of the ABP of the patient for one of a plurality of individual heartbeats of the patient;

determining (52), for each of the plurality of heartbeat portions, a set of coefficients representative of frequency components of the respective heartbeat portion to produce a plurality of sets of coefficients, each set of coefficients comprising a same number of coefficients;

normalizing (54) each set of coefficients to produce a plurality of sets of normalized coefficients;

determining a set of reference coefficients based on the plurality of sets of normalized coefficients;

comparing (60) a set of normalized coefficients for an individual heartbeat to the set of reference coefficients;

determining (62), based on the comparing, a quality indicator associated with the individual heartbeat;

using the quality indicator to produce (64) a modified hemodynamic sensor signal;

deriving one or more hemodynamic parameters from the modified hemodynamic sensor signal; and

outputting the derived hemodynamic parameters.

**15.** A memory (28), comprising

computer-readable code with instructions that, when executed by one or more processors of a hemodynamic monitor, cause the one or more processors to perform the following steps:

segregate (50) a received hemodynamic sensor signal into a plurality of heartbeat portions, each heartbeat portion representative of the arterial blood pressure, hereinafter ABP of the patient for one of a plurality of individual heartbeats of the patient;

determine (52), for each of the plurality of heartbeat portions, a set of coefficients representative of frequency components of the respective heartbeat portion to produce a plurality of sets of coefficients, each set of coefficients comprising a same number of coefficients;

normalize (54)each set of coefficients to produce a plurality of sets of normalized coefficients;

determine a set of reference coefficients based on the plurality of sets of normalized coefficients;

compare (60) a set of normalized coefficients for an individual heartbeat to the set of reference coefficients;

determine (62), based on the comparing, a quality indicator associated with the individual heartbeat;

use the quality indicator to produce (64) a modified hemodynamic sensor signal;

derive one or more hemodynamic parameters from the modified hemodynamic sensor signal; and

output the one or more derived hemodynamic parameters.

**Patentansprüche**

**1.** Hämodynamik-Monitor (12), umfassend:

eine Sensorschnittstelle (34), die ein hämodynamisches Sensorsignal von einem hämodynamischen Sensor (14, 16) empfängt, wobei das hämodynamische Sensorsignal den arteriellen Blutdruck, im Folgenden ABP genannt, eines Patienten (18) darstellt;

ein Herzschlag-Erkennungsmodul (40), das das empfangene hämodynamische Sensorsignal in eine Vielzahl von Herzschlagsabschnitten aufteilt, wobei jeder Herzschlagsabschnitt den ABP des Patienten für einen von mehreren individuellen Herzschlägen des Patienten darstellt;
ein Modellparameter-Modul (42), das:

für jeden der mehreren Herzschlagabschnitte einen Satz von Koeffizienten bestimmt, die für Frequenzkomponenten des jeweiligen Herzschlagabschnitts repräsentativ sind, um mehrere Sätze von Koeffizienten zu erzeugen, wobei jeder Satz von Koeffizienten dieselbe Anzahl von Koeffizienten umfasst; und
jeden Satz von Koeffizienten normalisiert, um mehrere Sätze von normalisierten Koeffizienten zu erzeugen;
ein Herzschlag-Klassifizierungsmodul (44), das:

einen Satz von Referenzkoeffizienten basierend auf den mehreren Sätzen von normalisierten Koeffizienten bestimmt; und
einen Qualitätsindikator bereitstellt, der einem individuellen Herzschlag zugeordnet ist, basierend auf einem Vergleich eines Satzes von normalisierten Koeffizienten für den individuellen Herzschlag mit dem Satz von Referenzkoeffizienten; und
ein Hämodynamik-Verarbeitungsmodul (38), das:

den Qualitätsindikator verwendet, um ein modifiziertes hämodynamisches Sensorsignal zu erzeugen;
einen oder mehrere hämodynamische Parameter aus dem modifizierten hämodynamischen Sensorsignal ableitet; und
den einen oder die mehreren abgeleiteten hämodynamischen Parameter ausgibt.

2.  Hämodynamik-Monitor nach Anspruch 1, wobei der Qualitätsindikator den individuellen Herzschlag entweder als qualifiziert für die Verwendung in der nachgelagerten Verarbeitung oder als nicht qualifiziert für die Verwendung in der nachgelagerten Verarbeitung einstuft.

3.  Hämodynamik-Monitor nach Anspruch 1, wobei das Herzschlag-Klassifizierungsmodul (44) den Qualitätsindikator basierend auf einem Ausmaß erzeugt, um das der Satz normalisierter Koeffizienten für den individuellen Herzschlag von dem Satz von Referenzkoeffizienten abweicht.

4.  Hämodynamik-Monitor nach Anspruch 3, wobei das Herzschlag-Klassifizierungsmodul (44) das Ausmaß, um das der Satz der normalisierten Koeffizienten für den individuellen Herzschlag von dem Satz der Referenzkoeffizienten abweicht, als Vektornorm der Differenz zwischen dem Satz der normalisierten Koeffizienten für den individuellen Herzschlag und dem Satz der Referenzkoeffizienten bestimmt.

5.  Hämodynamik-Monitor nach Anspruch 1, wobei das Modellparameter-Modul (42) für jeden der mehreren Herzschlagabschnitte den Satz von Koeffizienten bestimmt, die für die Frequenzkomponenten des jeweiligen Herzschlagabschnitts repräsentativ sind, durch:

Identifizieren eines Zeitabschnitts des jeweiligen Herzschlagabschnitts für jeden der mehreren Herzschlagabschnitte; und
Bestimmen des Satzes von Koeffizienten für den jeweiligen Herzschlagabschnitt basierend auf dem identifizierten Zeitabschnitt des jeweiligen Herzschlagabschnitts.

6.  Hämodynamik-Monitor nach Anspruch 5, wobei das Modellparameter-Modul (42) den Satz von Koeffizienten für den jeweiligen Herzschlagabschnitt basierend auf dem identifizierten Zeitabschnitt des jeweiligen Herzschlagabschnitts unter Verwendung einer Fourier-Reihenentwicklung des jeweiligen Herzschlagabschnitts als Funktion des identifizierten Zeitabschnitts des jeweiligen Herzschlagabschnitts bestimmt.

7.  Hämodynamik-Monitor nach Anspruch 6, wobei das Modellparameter-Modul (42) den Satz von Koeffizienten unter Verwendung der Fourier-Reihenentwicklung des jeweiligen Herzschlagabschnitts als Funktion des identifizierten Zeitabschnitts des jeweiligen Herzschlagabschnitts gemäß der Gleichung bestimmt:

$$c_l[n] = \frac{1}{T[n]} \int_0^{T[n]} s_n(t) e^{i\frac{-2\pi l(t - t_s[n])}{T[n]}} dt$$

wobei n eine Indexzahl ist, die den jeweiligen Herzschlagsabschnitt innerhalb des hämodynamischen Sensorsignals identifiziert;

wobei *l* ein Index ist, der von einem negativen Wert eines höchsten Frequenzkoeffizienten bis zu einem positiven Wert des höchsten Frequenzkoeffizienten reicht;

wobei $c_l[n]$ der Satz von Koeffizienten für den jeweiligen Herzschlagabschnitt ist;

wobei $T[n]$ der identifizierte Zeitabschnitt des jeweiligen Herzschlagabschnitts ist;

wobei $s_n(t)$ das hämodynamische Sensorsignal ist; und

wobei $t_s[n]$ ein Startzeitpunkt des jeweiligen Herzschlagabschnitts innerhalb des hämodynamischen Sensorsignals ist.

8.  Hämodynamik-Monitor nach Anspruch 1, wobei das Modellparameter-Modul (42) jeden Satz von Koeffizienten normalisiert, um die mehreren Sätze von normalisierten Koeffizienten zu erzeugen, indem es jeden Koeffizienten aus einem jeweiligen Satz von Koeffizienten durch eine 2-Norm des jeweiligen Satzes von Koeffizienten dividiert.

9.  Hämodynamik-Monitor nach Anspruch 1, wobei das Herzschlag-Klassifizierungsmodul (44) den Satz von Referenzkoeffizienten basierend auf den mehreren Sätzen von normalisierten Koeffizienten unter Verwendung einer iterativen Schätzung für einen Mittelwert der Sätze von normalisierten Koeffizienten bestimmt.

10. Hämodynamik-Monitor nach Anspruch 9, wobei das Herzschlag-Klassifizierungsmodul (44) den Satz von Referenzkoeffizienten unter Verwendung der iterativen Schätzung für den Mittelwert der Sätze von normalisierten Koeffizienten gemäß der Gleichung bestimmt:

$$\bar{c}_s[n] = (1 - \alpha)\bar{c}_s[n-1] + \alpha c_s[n]$$

wobei $\overline{c}_s[n]$ der Satz von Referenzkoeffizienten für einen Herzschlagindex $n$ ist; und

wobei $\alpha$ ein iterativer Mittelungsparameter ist.

11. Hämodynamik-Monitor nach Anspruch 10, wobei $\alpha$ eine Zahl größer als Null und kleiner als Eins ist.

12. Hämodynamik-Monitor nach Anspruch 1, wobei der Qualitätsindikator den individuellen Herzschlag entweder als qualifiziert für die Verwendung in der nachgelagerten Verarbeitung oder als nicht qualifiziert für die Verwendung in der nachgelagerten Verarbeitung einstuft; und

wobei das Hämodynamik-Verarbeitungsmodul (38) den Qualitätsindikator verwendet, um das modifizierte hämodynamische Sensorsignal zu erzeugen, durch:

Erzeugen des modifizierten hämodynamischen Sensorsignals so, dass der individuelle Herzschlag in dem modifizierten hämodynamischen Sensorsignal enthalten ist, in Reaktion auf das Bestimmen, dass der Qualitätsindikator den individuellen Herzschlag als qualifiziert für die Verwendung in der nachgelagerten Verarbeitung einstuft; und

Erzeugen des modifizierten hämodynamischen Sensorsignals so, dass der individuelle Herzschlag nicht in dem modifizierten hämodynamischen Sensorsignal enthalten ist, in Reaktion auf das Bestimmen, dass der Qualitätsindikator den individuellen Herzschlag als nicht qualifiziert für die Verwendung in der nachgelagerten Verarbeitung einstuft.

13. System (10), umfassend:

einen hämodynamischen Sensor (14, 16), der dazu konfiguriert ist, den arteriellen Blutdruck, im Folgenden als ABP bezeichnet, eines Patienten zu messen; und

einen Hämodynamik-Monitor (12) nach einem der vorangehenden Ansprüche, der mit dem hämodynamischen Sensor verbunden ist.

14. Computerimplementiertes Verfahren, umfassend:

Erzeugen (48) eines analogen hämodynamischen Sensorsignals unter Verwendung eines hämodynamischen Sensors, das für den arteriellen Blutdruck, im Folgenden als ABP bezeichnet, eines Patienten repräsentativ ist;

Abtasten (48) des analogen hämodynamischen Sensorsignals mit einer definierten Abtastrate, um ein abgetastetes hämodynamisches Sensorsignal zu erzeugen, das für den ABP des Patienten repräsentativ ist;

Aufteilen (50) des abgetasteten hämodynamischen Sensorsignals in mehrere Herzschlagabschnitte, wobei jeder Herzschlagabschnitt für den ABP des Patienten für einen von mehreren individuellen Herzschlägen des Patienten repräsentativ ist;

Bestimmen (52), für jeden der mehreren Herzschlagabschnitte, eines Satzes von Koeffizienten, die für Frequenzkomponenten des jeweiligen Herzschlagabschnitts repräsentativ sind, um mehrere Sätze von Koeffizienten zu erzeugen, wobei jeder Satz von Koeffizienten dieselbe Anzahl von Koeffizienten umfasst;

Normalisieren (54) jedes Satzes von Koeffizienten, um mehrere Sätze von normalisierten Koeffizienten zu erzeugen;

Bestimmen eines Satzes von Referenzkoeffizienten basierend auf den mehreren Sätzen von normalisierten Koeffizienten;

Vergleichen (60) eines Satzes von normalisierten Koeffizienten für einen individuellen Herzschlag mit dem Satz von Referenzkoeffizienten;

Bestimmen (62), basierend auf dem Vergleich, eines Qualitätsindikators, der dem individuellen Herzschlag zugeordnet ist;

Verwenden des Qualitätsindikators zum Erzeugen (64) eines modifizierten hämodynamischen Sensorsignals;

Ableiten eines oder mehrerer hämodynamischer Parameter aus dem modifizierten hämodynamischen Sensorsignal; und

Ausgeben der abgeleiteten hämodynamischen Parameter.

15. Speicher (28), der der computerlesbaren Code mit Anweisungen umfasst, die, wenn sie von einem oder mehreren Prozessoren eines Hämodynamik-Monitors ausgeführt werden, den einen oder die mehreren Prozessoren veranlassen, die folgenden Schritte durchzuführen:

Aufteilen (50) eines empfangenen hämodynamischen Sensorsignals in mehrere Herzschlagabschnitte, wobei jeder Herzschlagsabschnitt den arteriellen Blutdruck, im Folgenden als ABP bezeichnet, des Patienten für einen von mehreren individuellen Herzschlägen des Patienten repräsentiert;

Bestimmen (52), für jeden der mehreren Herzschlagabschnitte, eines Satzes von Koeffizienten, die für Frequenzkomponenten des jeweiligen Herzschlagabschnitts repräsentativ sind, um mehrere Sätze von Koeffizienten zu erzeugen, wobei jeder Satz von Koeffizienten dieselbe Anzahl von Koeffizienten umfasst;

Normalisieren (54) jedes Satzes von Koeffizienten, um mehrere Sätze von normalisierten Koeffizienten zu erzeugen;

Bestimmen eines Satzes von Referenzkoeffizienten basierend auf den mehreren Sätzen von normalisierten Koeffizienten;

Vergleichen (60) eines Satzes von normalisierten Koeffizienten für einen individuellen Herzschlag mit dem Satz von Referenzkoeffizienten;

Bestimmen (62), basierend auf dem Vergleich, eines Qualitätsindikators, der dem individuellen Herzschlag zugeordnet ist;

Verwenden des Qualitätsindikators zum Erzeugen (64) eines modifizierten hämodynamischen Sensorsignals;

Ableiten eines oder mehrerer hämodynamischer Parameter aus dem modifizierten hämodynamischen Sensorsignal; und

Ausgeben des einen oder der mehreren abgeleiteten hämodynamischen Parameter.

## Revendications

1. Moniteur hémodynamique (12) comprenant :

une interface (34) de capteur qui reçoit un signal de capteur hémodynamique provenant d'un capteur hémodynamique (14, 16), le signal de capteur hémodynamique étant représentatif de la tension artérielle, ci-après TA, d'un patient (18) ;

un module de détection de battement (40) qui sépare le signal de capteur hémodynamique reçu en une pluralité de parties de battement cardiaque, chaque partie de battement cardiaque étant représentative de la TA du patient pour un battement parmi une pluralité de battements cardiaques individuels du patient ;

un module de paramètre de modèle (42) qui :

détermine, pour chaque partie de la pluralité de parties de battement cardiaque, un ensemble de coefficients représentatifs de composantes de fréquence de la partie de battement cardiaque respective afin de produire une pluralité d'ensembles de coefficients, chaque ensemble de coefficients comprenant un même nombre de coefficients ; et

normalise chaque ensemble de coefficients afin de produire une pluralité d'ensembles de coefficients normalisés ;

un module de classification de battements cardiaques (44) qui :

détermine un ensemble de coefficients de référence sur la base de la pluralité d'ensembles de coefficients normalisés ; et

fournit un indicateur de qualité associé à un battement cardiaque individuel sur la base d'une comparaison d'un ensemble de coefficients normalisés pour le battement cardiaque individuel à l'ensemble de coefficients de référence ; et

un module de traitement hémodynamique (38) qui :

utilise l'indicateur de qualité afin de produire un signal de capteur hémodynamique modifié ;

dérive un ou plusieurs paramètres hémodynamiques à partir du signal de capteur hémodynamique modifié ; et

délivre en sortie ledit un ou lesdits plusieurs paramètres hémodynamiques dérivés.

2. Moniteur hémodynamique selon la revendication 1, dans lequel l'indicateur de qualité classifie le battement cardiaque individuel comme étant soit qualifié pour une utilisation dans un traitement en aval soit non qualifié pour une utilisation dans le traitement en aval.

3. Moniteur hémodynamique selon la revendication 1, dans lequel le module de classification de battements cardiaques (44) génère l'indicateur de qualité sur la base d'une étendue par laquelle l'ensemble de coefficients normalisés pour le battement cardiaque individuel s'écarte de l'ensemble de coefficients de référence.

4. Moniteur hémodynamique selon la revendication 3, dans lequel le module de classification de battements cardiaques (44) détermine l'étendue par laquelle l'ensemble de coefficients normalisés pour le battement cardiaque individuel s'écarte de l'ensemble de coefficients de référence en tant que norme vectorielle de la différence entre l'ensemble de coefficients normalisés pour le battement cardiaque individuel et l'ensemble de coefficients de référence.

5. Moniteur hémodynamique selon la revendication 1, dans lequel le module de paramètre de modèle (42) détermine, pour chaque partie de la pluralité de parties de battement cardiaque, l'ensemble de coefficients représentatifs de composantes de fréquence de la partie de battement cardiaque respective par :

l'identification, pour chaque partie de la pluralité de parties de battement cardiaque, d'une période de la partie de battement cardiaque respective ; et

la détermination de l'ensemble de coefficients pour la partie de battement cardiaque respective sur la base de la période identifiée de la partie de battement cardiaque respective.

6. Moniteur hémodynamique selon la revendication 5, dans lequel le module de paramètre de modèle (42) détermine l'ensemble de coefficients pour la partie de battement cardiaque respective sur la base de la période identifiée de la partie de battement cardiaque respective à l'aide d'un développement en série de Fourier de la partie de battement cardiaque respective en fonction de la période identifiée de la partie de battement cardiaque respective.

7. Moniteur hémodynamique selon la revendication 6, dans lequel le module de paramètre de modèle (42) détermine l'ensemble de coefficients à l'aide du développement en série de Fourier de la partie de battement cardiaque respective en fonction de la période identifiée de la partie de battement cardiaque respective selon l'équation :

$$c_l[n] = \frac{1}{T[n]} \int_0^{T[n]} s_h(t) e^{\frac{-2\pi l(t - t_s[n])}{T[n]}} dt$$

dans laquelle $n$ représente un numéro d'index identifiant la partie de battement cardiaque respective au sein du signal de capteur hémodynamique ;

dans laquelle $l$ représente un indice allant d'une valeur négative d'un coefficient de fréquence le plus élevé à une valeur positive du coefficient de fréquence le plus élevé ;

dans laquelle $c_l[n]$ représente l'ensemble de coefficients pour la partie de battement cardiaque respective ;

dans laquelle *T[n]* représente la période identifiée de la partie de battement cardiaque respective ;

dans laquelle $s_n(t)$ représente le signal de capteur hémodynamique ; et

dans laquelle $t_s[n]$ représente une heure de début au sein du signal de capteur hémodynamique de la partie de battement cardiaque respective.

8. Moniteur hémodynamique selon la revendication 1, dans lequel le module de paramètre de modèle (42) normalise chaque ensemble de coefficients afin de produire la pluralité d'ensembles de coefficients normalisés en divisant chaque coefficient provenant d'un ensemble respectif de coefficients par une norme euclidienne de l'ensemble respectif de coefficients.

9. Moniteur hémodynamique selon la revendication 1, dans lequel le module de classification de battements cardiaques (44) détermine l'ensemble de coefficients de référence sur la base de la pluralité d'ensembles de coefficients normalisés à l'aide d'une estimation itérative pour une moyenne des ensembles de coefficients normalisés.

10. Moniteur hémodynamique selon la revendication 9, dans lequel le module de classification de battements cardiaques (44) détermine l'ensemble de coefficients de référence à l'aide de l'estimation itérative pour la moyenne des ensembles de coefficients normalisés selon l'équation :

$$\bar{c}_s[n] = (1 - \alpha)\bar{c}_s[n-1] + \alpha c_s[n]$$

dans laquelle $\bar{c}_s[n]$ représente l'ensemble de coefficients de référence pour un index de battement cardiaque n ; et

dans laquelle $\alpha$ représente un paramètre de moyennage itératif.

11. Moniteur hémodynamique selon la revendication 10, dans lequel $\alpha$ représente un nombre supérieur à zéro et inférieur à un.

12. Moniteur hémodynamique selon la revendication 1, dans lequel l'indicateur de qualité classifie le battement cardiaque individuel comme étant soit qualifié pour une utilisation dans un traitement en aval ou soit non qualifié pour une utilisation dans le traitement en aval ; et

dans lequel le module de traitement hémodynamique (38) utilise l'indicateur de qualité afin de produire le signal de capteur hémodynamique modifié par :

la production du signal de capteur hémodynamique modifié afin d'inclure le battement cardiaque individuel dans le signal de capteur hémodynamique modifié en réponse à la détermination du fait que l'indicateur de qualité classifie le battement cardiaque individuel comme étant qualifié pour une utilisation dans le traitement en aval ; et

la production du signal de capteur hémodynamique modifié afin de ne pas inclure le battement cardiaque individuel dans le signal de capteur hémodynamique modifié en réponse à la détermination du fait que l'indicateur de qualité classifie le battement cardiaque individuel comme étant non qualifié pour une utilisation dans le traitement en aval.

13. Système (10), comprenant :

un capteur hémodynamique (14, 16) configuré pour détecter la tension artérielle, ci-après TA, d'un patient ; et

un moniteur hémodynamique (12) selon l'une des revendications précédentes relié au capteur hémodynamique.

14. Procédé mis en oeuvre par ordinateur, comprenant :

la production (48), à l'aide d'un capteur hémodynamique, d'un signal de capteur hémodynamique analogique représentatif de la tension artérielle, ci-après TA, d'un patient ;

l'échantillonnage (48) du signal de capteur hémodynamique analogique à une fréquence d'échantillonnage définie afin de produire un signal de capteur hémodynamique échantillonné représentatif de la TA du patient ;

la séparation (50) du signal de capteur hémodynamique échantillonné en une pluralité de parties de battement cardiaque, chaque partie de battement cardiaque étant représentative de la TA du patient pour un battement parmi une pluralité de battements cardiaques individuels du patient ;

la détermination (52), pour chaque partie de la pluralité de parties de battement cardiaque, d'un ensemble de coefficients représentatifs de composantes de fréquence de la partie de battement cardiaque respective afin de produire une pluralité d'ensembles de coefficients, chaque ensemble de coefficients comprenant un même nombre de coefficients ;

la normalisation (54) de chaque ensemble de coefficients afin de produire une pluralité d'ensembles de coefficients normalisés ;

la détermination d'un ensemble de coefficients de référence sur la base de la pluralité d'ensembles de coefficients normalisés ;

la comparaison (60) d'un ensemble de coefficients normalisés pour un battement cardiaque individuel à l'ensemble de coefficients de référence ;

la détermination (62), sur la base de la comparaison, d'un indicateur de qualité associé au battement cardiaque individuel ;

l'utilisation de l'indicateur de qualité afin de produire (64) un signal de capteur hémodynamique modifié ;

la dérivation d'un ou de plusieurs paramètres hémodynamiques à partir du signal de capteur hémodynamique modifié ; et

la délivrance en sortie des paramètres hémodynamiques dérivés.

15. Mémoire (28), comprenant

un code lisible par ordinateur comportant des instructions qui, lorsqu'elles sont exécutées par un ou plusieurs processeurs d'un moniteur hémodynamique, amènent ledit un ou lesdits plusieurs processeurs à effectuer les étapes suivantes :

séparation (50) d'un signal de capteur hémodynamique reçu en une pluralité de parties de battement cardiaque, chaque partie de battement cardiaque étant représentative de la tension artérielle, ci-après TA, du patient pour un battement parmi une pluralité de battements cardiaques individuels du patient ;

détermination (52), pour chaque partie de la pluralité de parties de battement cardiaque, d'un ensemble de coefficients représentatifs de composantes de fréquence de la partie de battement cardiaque respective afin de produire une pluralité d'ensembles de coefficients, chaque ensemble de coefficients comprenant un même nombre de coefficients ;

normalisation (54) de chaque ensemble de coefficients afin de produire une pluralité d'ensembles de coefficients normalisés ;

détermination d'un ensemble de coefficients de référence sur la base de la pluralité d'ensembles de coefficients normalisés ;

comparaison (60) d'un ensemble de coefficients normalisés pour un battement cardiaque individuel à l'ensemble de coefficients de référence ;

détermination (62), sur la base de la comparaison, d'un indicateur de qualité associé au battement cardiaque individuel ;

utilisation de l'indicateur de qualité afin de produire (64) un signal de capteur hémodynamique modifié ;

dérivation d'un ou de plusieurs paramètres hémodynamiques à partir du signal de capteur hémodynamique modifié ; et

délivrance en sortie dudit un ou desdits plusieurs paramètres hémodynamiques dérivés.

Fig. 1

EP 4 033 969 B1

```
┌─────────────────────────────────────────────────────────────────────────────────┐
│  ┌──────────────────┐      ┌──────────────────┐      ┌──────────────────┐        │
│  │  Beat Detection  │      │ Model Parameter  │      │ Beat Classification │     │
│  │     Module       │──────▶│     Module      │──────▶│      Module       │──────▶│
│  │                  │      │                  │      │                  │        │
│  │       40         │      │       42         │      │       44         │        │
│  └──────────────────┘      └──────────────────┘      └──────────────────┘        │
└─────────────────────────────────────────────────────────────────────────────────┘
```

Fig. 2

Fig. 3

| Receive Heartbeat Portion |—— 48 |

↓

| Determine Period of Heartbeat Portion |—— 50 |

↓

| Determine Set of Frequency Component Coefficients for Heartbeat Portion |—— 52 |

↓

| Normalize Set of Frequency Component Coefficients for Heartbeat Portion |—— 54 |

↓

| Provide Normalized Set of Frequency Component Coefficients for Heartbeat Portion |—— 56 |

# Fig. 4

```
┌─────────────────────────────────────┐
│  Receive Normalized Set of Frequency │
│  Component Coefficients for Individual│──── 58
│         Heartbeat Portion            │
└─────────────────────────────────────┘
                  │
                  ▼
┌─────────────────────────────────────┐
│   Compare Normalized Set of Frequency│
│ Component Coefficients to Set of     │──── 60
│        Reference Coefficients        │
└─────────────────────────────────────┘
                  │
                  ▼
┌─────────────────────────────────────┐
│  Provide Quality Indicator for the   │
│    Individual Heartbeat Portion      │
│   Based Upon the Comparison of the   │
│   Normalized Set of Frequency        │──── 62
│  Component Coefficients to the       │
│    Set of Reference Coefficients     │
└─────────────────────────────────────┘
                  │
                  ▼
┌─────────────────────────────────────┐
│  Update the Reference Set of         │
│  Coefficients Based on the Quality   │──── 64
│            Indicator                 │
└─────────────────────────────────────┘
```

Fig. 5

Receive Normalized Set of Frequency
Component Coefficients for Individual
Heartbeat Portion ——66

Generate Reference Set of Coefficients
Using the Normalized Set of Frequency
Component Coefficients ——68

70

Initial Learning
Period Criteria
Satisfied ?

No

Yes

Exit Learning Period ——72

Fig. 6